Europäisches Patentamt

(19) European Patent Office

Office européen des brevets

(11) **EP 0 819 434 B1**

(12) **EUROPÄISCHE PATENTSCHRIFT**

(45) Veröffentlichungstag und Bekanntmachung des Hinweises auf die Patenterteilung:
**17.10.2001  Patentblatt 2001/42**

(51) Int Cl.7: **A61K 31/785**, A61K 31/155

(21) Anmeldenummer: **97111803.9**

(22) Anmeldetag: **11.07.1997**

(54) **Verwendung von PHMB zur Behandlung von Tumorerkrankungen**

Use of PHMB for the treatment of tumors

Utilisation du PHMB pour le traitement de tumeurs

(84) Benannte Vertragsstaaten:
**DE ES FR GB IT**

(30) Priorität: **16.07.1996  DE 19628641**

(43) Veröffentlichungstag der Anmeldung:
**21.01.1998  Patentblatt 1998/04**

(73) Patentinhaber: **Fresenius AG**
**61350 Bad Homburg v.d.H (DE)**

(72) Erfinder:
• **Kirschner, Ulrich, Dr.**
**61350 Bad Homburg v.d.H. (DE)**
• **Jethon, Frank**
**61350 Bad Homburg v.d.H. (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**GB-A- 2 134 783**

• **DATABASE WPI Section Ch, Week 9434 Derwent Publications Ltd., London, GB; Class B05, AN 94-277479 XP002047067 & RU 2 008 001 C (BLESKIN B I) , 28.Februar 1994**
• **BAUMANIS, E.A., BIRSKA I.A. ET AL.: "Cytological and immunological aspects of antitumor action of polyhexamethylene guanidine in BALB/C mice" EKSP. ONKOL., Bd. 11, Nr. 3, 1989, USSR, Seiten 66-69, XP002047066**

**Beschreibung**

Gegenstand der vorliegenden Erfindung ist die Verwendung von Poly(hexamethylen)biguanid zur Zubereitung eines Medikaments zur Behandlung von Tumorerkrankungen.

Aus dem Stand der Technik ist bekannt, daß Poly(hexamethylen)biguanid (PHMB) eine bakterizide und fungizide Wirkung aufweist (vgl. z.B. GB-PS 1,202,495). PHMB wird daher in vielen Bereichen als Desinfektionsmittel, z. B. in Form von Lösungen oder Spray's eingesetzt. Anwendung finden sie z.B. in der Lebensmittelindustrie zur Reinigung und Desinfektion von Räumen und Geräten, zur Stabilisierung von Getränken und zur Reinigung und Stabilisierung von Wasser, z.B. auch in Schwimmbädern zur Bekämpfung von Algen- und Bakterienwuchs. Aus der DE-OS 35 37 627 ist bekannt, daß durch Kombination von PHMB mit einem Molekulargewicht von 1.700 bis 2.500 mit einer geringen Menge Polyethylenglycol Desinfektionsmittel erhalten werden, die auch als lokale Antiseptika in der Wundbehandlung eingesetzt werden. Als PHMB geeignet ist gemäß dieser DE-OS z.B. das PHMB, das als Hydrochlorid unter dem Warenzeichen Vantocil® IB von der ICI vertrieben wird.

In der EP 0 4 50 117 wird eine Ringerlösung und deren Anwendung als bakterizid wirkendes lokales Wundbehandlungsmedikament beschrieben, wobei die lactatfreie Ringerlösung zusätzlich 0,1 % bis 0,2 % eines Konzentrates gelöst enthält, das aus einer 20 %igen wässrigen Poly(hexamethylen)biguanidhydrochlorid-Lösung besteht, in der pro 100 ml 1 Polyethylenglycol mit einem Molekulargewicht von etwa 4.000 gelöst ist. Als PHMB wird ebenfalls lediglich das unter dem Warenzeichen Vantocil® IB der Firma ICI vertriebene Produkt als geeignet beschrieben. Unter dem Warenzeichen Lavasept® ist ein Produkt für die Wundheilung bekannt, wobei das Lavasept-Konzentrat eine wässrige Lösung von 20 Gew.-% PHMB und 1 Gew.-% Polyethylenglycol 4.000 darstellt und das PHMB das Handelsprodukt Vantocil® IB der Firma ICI ist.

In der US-PS 4,758,591 werden Lösungen beschrieben, die eine mikrobizid- oder fungizid-wirksame Menge eines Biguanides oder eines wasserlöslichen Salzes desselben in einer Menge von 0,000001 bis 0,0003 Gew.-% enthalten, die für Kontaktlinsen, ophthalmologische Produkte und dermatologische Formulierungen, die nahe des Auges angewandt werden, eingesetzt werden.

Aus der GB-PS 1,432,345 sind Zusammensetzungen für die Verwendung in Verbindung mit Augen und Kontaktlinsen bekannt, die mindestens ein ophthalmisch akzeptables polymeres Biguanid enthalten.

Tumorerkrankungen zählen nach wie vor zu den häufigsten Erkrankungen und es wurden bereits zahlreiche Mittel vorgeschlagen, die zu deren Behandlung mit unterschiedlichem Erfolg eingesetzt wurden.

Die Synthese und Entwicklung neuer antitumoraler Substanzen sowie das Screening unter bereits bekannten Substanzen nach solchen, die antitumorale Eigenschaften besitzen, ist ein Hauptanliegen der heutigen Krebsforschung.

Es besteht nach wie vor der Bedarf nach geeigneten Mitteln, mit denen Tumore bzw. Tumorerkrankungen wirksam behandelt werden können.

Die Aufgabe der vorliegenden Erfindung ist es daher, ein Mittel bereitzustellen, mit dem Tumorerkrankungen wirksamer und schneller therapiert werden können. Insbesondere soll ein Mittel bereitgestellt werden, das lokal und gegebenenfalls systemisch einsetzbar ist und eine hohe Wirksamkeit gegen Tumore besitzt, ohne daß die Zellen des umgebenden Gewebes zerstört werden.

Erfindungsgemäß wurde überraschend gefunden, daß diese Aufgabe durch die Verwendung von Poly(hexamethylen)biguanid (PHMB) gelöst werden kann. PHMB kann das Wachstum verschiedener Tumorarten hemmen. Ferner wurde erfindungsgemäß überraschend gefunden, daß Poly(hexamethylen)biguanid auf bestimmte Tumortypen, insbesondere Kolonkarzinome, eine selektive Wirkung besitzt. Unter den für PHMB sensiblen Tumorarten befinden sich auch solche, die gegen andere bekannte Antitumorwirkstoffe (vgl. z.B. Eur J Cancer Clin Oncol 1987, 123:937-948; Fiebig, Berger, (eds.) Immunodeficient Mice in Oncology, Contrib. Oncol. Basel, Karger, 1992, Vol 42, 321-351: Med Welt 1984; 35:52-58, 81-86) eine hohe Resistenz aufweisen.

Bevorzugt ist erfindungsgemäß ein Poly(hexamethylen)biguanid mit einer mittleren Molekulargewichtsverteilung bis 15.000.

Besonders bevorzugt ist ein Poly(hexamethylen)biguanid mit einer mittleren Molekulargewichtsverteilung von 1.000 bis 8.000 und insbesondere ein PHMB mit einer mittleren Molekulargewichtsverteilung von 1.700 bis 5.000, z. B. mit einem mittleren Molekulargewicht $M_W$ von 2.600, von 2.800, von 3.500, von 4.000 oder von 4.500.

Die Molekulargewichtsbestimmung erfolgt auf viskosimetrischem Wege.

Die Herstellung des erfindungsgemäß verwendeten Poly(hexamethylen)biguanids erfolgt in an sich bekannter Weise, z.B. wie es in der DE-PS 16 20 938 oder der GB-PS 1,202,495, auf deren Offenbarung hiermit im vollen Umfang Bezug genommen wird, beschrieben ist. Aus dem erhaltenen Poly(hexamethylen)biguanid kann gegebenenfalls in an sich bekannter Weise ein unerwünschter Molekulargewichtsanteil, z.B. durch Dialyse, Molekularfiltration, HPLC, Gel-Permeations-Chromatographie (GPC), fraktionierte Fällung und dergleichen abgetrennt werden. Beispielsweise können auf diese Weise die niedermoleklaren Bestandteile unter Erzielung eines PHMB mit geringerer Toxizität bei gleichgroßer Wirksamkeit aus dem handelsüblichen PHMB entfernt werden. In diesem Zusammenhang wird auf die PCT/EP94/01587 verwiesen, auf die zu Offenbarungszwecken hier in vollem Umfang Bezug genommen wird.

Erfindungsgemäß geeignet sind handelsübliches PHMB, wie z.B. Vantocil® IB, Cosmocil CQ oder Arlagard® E oder andere Handelsprodukte, aus denen gegebenenfalls die unerwünschte toxische niedermolekulare Fraktion, wie vorstehend beschrieben, abgetrennt wurde.

Das erfindungsgemäß eingesetzte PHMB liegt in freier Form oder in Form eines Salzes, wie eines wasserlöslichen Salzes, z.B. als Hydrochlorid, als Pulver (z.B. gefriergetrocknet) in 100 %iger Konzentration oder in wässriger Lösung vor. Es ist in Konzentrationen bis 40 Gew.-%, z.B. in 2 bis 40 Gew.-%, vorzugsweise 3 bis 30 Gew.-%, insbesondere 4 bis 20 Gew.-%, z.B. 4 Gew.-%, 4,5 Gew.-%, 5 Gew.-%, 6 Gew.-% in der wässrigen Lösung (d.h. als Konzentrat) verfügbar.

Unter "PHMB" wird hierin sowohl das Poly(hexamethylen)biguanid als auch das Poly(hexamethylen)biguanid in Form eines Salzes, z.B. eines wasserlöslichen Salzes wie des Hydrochlorids, verstanden.

Die Konzentration, in der das erfindungsgemäß verwendete PHMB bzw. die wässrige Lösung des PHMB zur Behandlung von Tumoren bzw. Tumorerkrankungen verwendet wird, hängt von dem beabsichtigten Verwendungszweck ab. Geeignete Konzentrationen liegen im allgemeinen im Bereich von 0,0001 bis 1 Gew.-%, vorzugsweise im Bereich von 0,0005 bis 0,1 Gew.-%, insbesondere im Bereich von 0,001 bis 0,04 Gew.-%, beispielsweise 0,01 oder 0,03 Gew.-% (bezogen auf PHMB).

Das erfindungsgemäß verwendete PHMB kann zusammen mit die Oberflächenspannung herabsetzenden Tensiden, wie z.B. Polyethylengycol verwendet werden. Vorzugsweise wird Polyethylenglycol mit einem Molekulargewicht von 1.500 bis 6.000 und insbesondere ein solches Polyethylenglycol mit einem Molekulargewicht von 4.000, wie es unter dem Warenzeichen Lutrol® E 4000 von der Firma BASF AG vertrieben wird, angewandt. Das Verhältnis von PHMB zu dem Tensid liegt geeigneterweise im Bereich von 6:1 bis 24:1, vorzugsweise im Bereich von 12:1 bis 22:1 und beträgt insbesondere 20:1.

Das erfindungsgemäß verwendete PHMB ist gegenüber Tumoren, wie z.B. Kolontumoren, Melanomen, Renaltumoren, Ovarialtumoren, Lungentumoren, Mammatumoren, Pankreastumoren und dergleichen in hohem Maße wirksam. Besonders wird es zur Behandlung von Kolontumoren, Lungentumoren, Melanomen, Renaltumoren und Mammatumoren angewandt. Insbesondere erfolgt erfindungsgemäß die Behandlung von Kolontumoren.

Die Behandlung erfolgt erfindungsgemäß lokal oder systemisch, z.B. oral, rektal, vaginal oder durch parenterale Verabreichung, vorzugsweise jedoch lokal.

Abhängig von der vorgesehenen Behandlung kann die Verwendung z.B. in Form von Tropfen, von wässrigen Lösungen (gegebenenfalls mit einem Gehalt an lactatfreier Ringerlösung oder Kochsalzlösung, vorzugsweise lactatfreier Ringerlösung), Emulsionen, Suspensionen, Gelen, Salben, Pasten, Cremes, Dragees oder Tabletten erfolgen. Spezielle Beispiele sind

Lösungen zur intratumoralen Injektion, z.B. bei Hautkrebsformen, wie Melanomen,

Gele, Salben, Pasten zur äußerlichen Anwendung, z.B. bei Hautkrebsformen, wie Melanomen,

Lösungen zur intraperitonealen Spülung, z.B. bei in den Peritonealraum metastasierenden Karzinomformen (z.B. Kolonkarzinomen),

Lösungen für Kolonspülungen,

Lösungen für Urethralspülungen,

magensaftresistente Darreichungsformen, wie z.B. Dragees, die die Freisetzung des Wirkstoffes z.B. selektiv im Darmtrakt erlauben,

Darreichungsformen, die die Freisetzung des Wirkstoffes z.B. selektiv im Magen erlauben,

i.v.-Lösungen, mit speziell für diese Anwendung aufbereiteter PHMB-Fraktion (d.h. PHMB, aus dem die niedermolekularen Molekulargewichtsanteile entfernt wurden, z.B. PHMB mit mittlerem Molekulargewicht von 2900 bis 15000, wie in der PCT/EP94/01587 beschrieben).

In diesen Zubereitungsformen sind gegebenenfalls zusätzlich die zur Herstellung der jeweiligen Zubereitungsform notwendigen üblichen Hilfs- und Zusatzstoffe enthalten.

Die Anwendung kann bei Mensch und Tier, vorzugsweise bei Menschen erfolgen.

Die nachfolgenden Beispiele dienen der Erläuterung der vorliegenden Erfindung.

**Beispiel 1**

Aus Poly(hexamethylen)biguanid-Hydrochlorid mit einem mittleren Molekulargewicht $M_w$ von 2.800, Polyethylenglycol 4.000 (Lutro® E 4000, BASF AG) und Wasser wurde durch Vermischen derselben eine Lösung der folgenden Zusammensetzung hergestellt:

| | |
|---|---|
| Poly(hexamethylen)biguanid-Hydrochlorid, $M_w$ 2.800 | 20 Gew.-% |
| Polyethylenglycol, $M_w$ 4.000 | 1 Gew.-% |
| Wasser | 79 Gew.-%. |

Die hergestellte Lösung wurde nach entsprechender Verdünnung erfolgreich zur Behandlung von Tumoren angewandt.

Als Poly(hexamethylen)biguanid-Hydrochlorid wurde das handelsübliche Poly(hexamethylen)biguanid-Produkt Vantocil® IB, Cosmocil CQ oder Arlagard® E der Firma ICI eingesetzt. Vantocil® IB, Cosmocil CQ bzw. Arlagard® E ist eine wässrige Lösung, die als Wirkstoff 20 % Poly(hexamethylen)biguanid--Hydrochlorid enthält.

**Beispiel 2**

Zur Herstellung einer weiteren Lösung für die erfindungsgemäße Verwendung wurde Beispiel 1 wiederholt mit der Ausnahme, daß anstelle des PHMB-Hydrochlorids das entsprechende PHMB eingesetzt wurde.

**Beispiel 3**

Untersuchung der antitumoralen Wirksamkeit von PHMB-Hydrochlorid, $M_w$ 2.800.

Die Untersuchung wurde unter Verwendung der Lösung gemäß Beispiel 1 in folgender Weise durchgeführt:

Die Untersuchung wurde an den in Tabelle 1 aufgeführten Tumorarten, deren Eigenschaften durch die Literatur (z.B. Berger et al.: Establishment and Characterization of Human Tumor Xenografts in thymusaplastic nude mice. In: Fiebig, Berger (eds) Immunodeficient Mice in Oncology. Contrib. Oncol. Basel, Karger, 1992; Fiebig, Berger (eds.) Immunodeficient Mice in Oncology. Contrib. Oncol. Basel, Karger, 1992, Vol. 42, S. 97-97 und Fiebig, Berger (eds.) Immunodeficient Mice in Oncology, Contrib. Oncol. Basel, Karger, 1992, Vol. 42, S. 148-151) charakterisiert sind, durchgeführt.

Tabelle 1

| "Tumorarten" | |
|---|---|
| **Name** | **Herkunft** |
| CXF 280 | Kolon |
| CXF 609 | Kolon |
| CXF HT29X | Kolon |
| LXFA 289 | Lunge |
| LXFA 526 | Lunge |
| LXFE 409 | Lunge |
| LXFL 529 | Lunge |
| LXFS 538 | Lunge (small cell) |
| LXFS 650 | Lunge (small cell) |
| MAXF MCF7X | Mamma |
| MEFX 514 | Melanom |
| MEFX 989 | Melanom |
| OVXF 1023 | Ovar |
| PRXF PC3M | Pankreas |
| RXF 486 | Renal |
| RXF 1220 | Renal |
| RXF 944LX | Renal |

Die in-vitro Aktivität von PHMB gegen die Stammzell-Fraktion der jeweiligen Tumorart wurde nach einem von Fiebig et al. (Eur J Cancer Clin Oncol 1987, 123:937-948; Fiebig, Berger, (eds.) Immunodeficient Mice in Oncology, Contrib. Oncol. Basel, Karger, 1992, Vol 42, 321-351: Med Welt 1984; 35:52-58, 81-86) modifizierten Protokoll des Zwei-Lagen-Weichagar-Kulturverfahrens (two-layer soft agar culture system) nach Hamburger und Salmon (Science 197: 461-463, 1977) untersucht. Die einzelnen Versuchsschritte sind nachfolgend beschrieben.

Festes Tumormaterial wurde in kleingeschnittenen Stücken in einem Stomacher vorsichtig mechanisch desintegriert und mit einem Enzym-Cocktail aus 0,05 % Collagenase, 0,07 % DNAse und 0,1 % Hyaluronidade für 30 min bei 37°C inkubiert. Anschließend wurden die Zellen zweimal mit Zellkulturmedium gewaschen und nacheinander durch Siebe mit 200 μm und 50 μm Maschenweite passagiert. Die Bestimmung des Anteils verfügbarer Zellen wurde in einem Hämocytometer mit der Trypan Blau Ausschlußmethode (lebende Zellen nehmen keinen Farbstoff auf) durchgeführt.

Die Tumorzellsuspension wurde in Multititerplatten mit 24 Wells über einer Bodenschicht, die 0,2 ml Iscoves Medium mit 20 % fetalem Kälberserum und 0,7 % Agar enthielt, ausplatiert. Dazu wurden 20.000 bis 200.000 Zellen zu 0,2 ml desselben Zellkulturmediums mit 0,4 %Agar gegeben und auf der Bodenschicht ausplatiert. Durch den geringen Agaranteil entsteht eine schwammartige Struktur des Mediums, die ein dreidimensionales Wachstum der Tumorkolonien ermöglicht. Die verschiedenen Konzentrationen der Prüfsubstanz wurden in Dreifach-Bestimmung unter kontinuierlicher Exposition (Drug Overlay Methode) in 0,2 ml Zellkulturmedium gegen eine Kontrolle ohne Prüfsubstanz untersucht. Die Kulturen wurden für 6-18 Tage bei 37°C und 7 % $CO_2$ in mit Luftfeuchte gesättigter Atmosphäre inkubiert.

Die Untersuchung des Koloniewachstums erfolgte in den Kontrollansätzen ohne Wirksubstanz mit einem inversen Mikroskop. Über die Dauer der Inkubation bilden sich durch in vitro Wachstum Kolonien mit einem Durchmesser > 50 μm heran. Zum Zeitpunkt des größten Koloniewachstums wurden die Multititerplatten mit einem automatischen Bildanalysesystem (Bausch und Lomb, Omnicom FAS IV) ausgewertet. Zur besseren Kontrastierung wurden die Kolonien 24 Stunden vor der Auswertung mit Tetrazoliumchlorid gefärbt.

Behandlung der Testkulturen mit der Prüfsubstanz

Geprüft wurden verschiedene Konzentrationen des PHMB-Hydrochlorid ($M_w$ 2.800) in den Abstufungen 10, 30, 100, 300 und 1000 μg/ml. Die Inkubationszeit betrug je nach Proliferationszeit 5-18 Tage. Die Prüfsubstanz wurde dreifach konzentriert mit Nährmedium verdünnt als oberste Schicht in die Wells der Mikrotiterplatten gegeben. Dadurch stellte sich in der Bodenschicht und der Tumorzellschicht durch Diffusion die gewünschte Endkonzentration der Prüfsubstanz ein.

Zur Wachstumskontrolle wurden Ansätze ohne Testsubstanz in jedem Versuch mitgeführt. Als Positivkontrolle wurden Ansätze mit der Referenzsubstanz 5-FU mitgeführt.

Zur Berechnung der Wirksamkeit der Prüfsubstanz wurde die Anzahl der überlebenden Zellkolonien der verschiedenen Tumorarten in Prozent bestimmt, indem die Koloniezahl in den behandelten Versuchsansätzen (T = Test) mit der Koloniezahl im Kontrollansatz (C = Kontrolle) nach der folgenden Proportion in Relation gesetzt wurde:

$$T/C = \text{Koloniezahl}_{\text{Kontrolle unbehandelt}} \times 100/\text{Koloniezahl}_{\text{Kontrollansatz}}$$

Ein Test wurde als vollständig auswertbar eingestuft, wenn die folgenden Qualitätskriterien erfüllt waren:

a) Anzahl der Kolonien in den Kontrollansätzen mit einem Durchmesser von 50 μm > 20,
b) Ausgangskoloniezahl am Tag 0 bzw. 2 < 30 % der Endkoloniezahl in der Kontrolle,
c) Variationskoeffizient in der Kontrollgruppe < 50 %,
d) Überlebensrate mit der Referenzsubstanz 5-FU bei der toxischen Dosis von 1000 μg/ml < 30 % des Kontrollansatzes ohne Behandlung.

Bei dieser Untersuchung wurden die folgenden Ergebnisse erhalten:

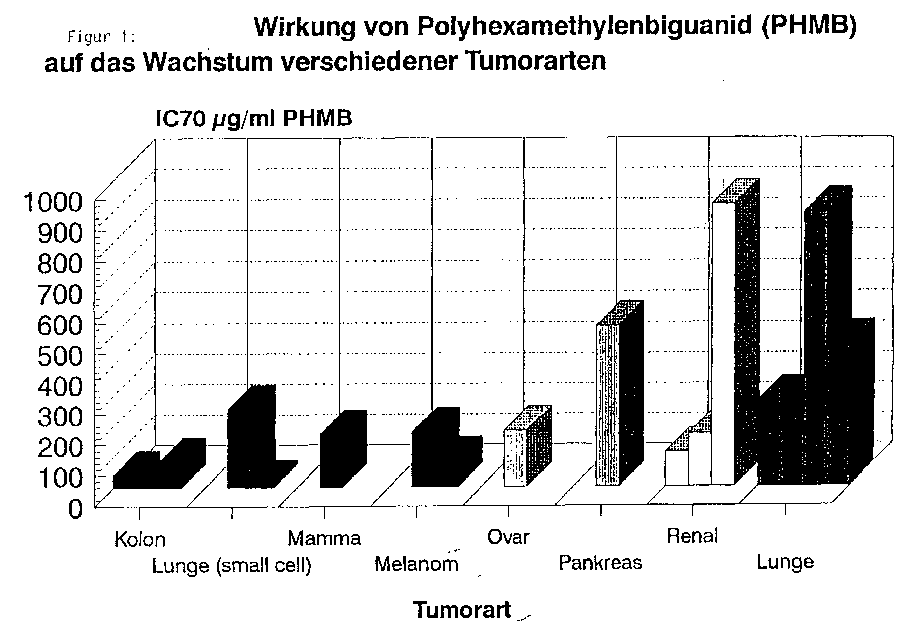

Das PHMB bewirkte in einer Konzentration von 300 μg/ml eine Hemmung des Koloniewachstums auf weniger als 30 % der Kontrolle (= wirksam) bei 13 von 17 geprüften Tumorarten. Bei einer Konzentration von 100 μg/ml wurden noch 5 der geprüften Tumorarten auf 30 % des Kontrollwachstums gehemmt. Die Ergebnisse sind in Tabelle 2 zusammengefaßt und in Abbildung 1 grafisch dargestellt.

Die Konzentrationen, bei denen eine Hemmung des Tumorwachstums auf 30 % des Kontrollwachstums erfolgte, sind als IC70-Werte (Konzentration in μg/ml bei der 70 % der Kolonien abgetötet werden) angegeben.

Die empfindlichsten Tumore waren die Kolonkarzimone CXF 280, CXF 609, HT29X, das Lungenkarzinom (small cell) LXFS 650, das Melanom MEFX 989, die Renalkarzinome RXF 486, RXF 1220 und das Mammakarzinom MCF7.

Tabelle 2

| Wirkung von Poly(hexamethylen)biguanid (PHMB) auf das Wachstum verschiedener Tumorarten anhand der IC 70-Werte | | |
|---|---|---|
| **Zellinie** | **Tumorart** | **IC70 μg/ml PHMB T/C < 30 %** |
| CXF 280 | Kolon | 41,485 |
| CXF 609 | Kolon | 13,687 |
| CXF HT29X | Kolon | 85,658 |
| LXFA 289 | Lunge | 283,145 |
| LXFA 526 | Lunge | 186,366 |
| LXFE 409 | Lunge | 897,583 |
| LXFL 529 | Lunge | 468,224 |
| LXFS 538 | Lunge (small cell) | 256,425 |
| LXFS 650 | Lunge (small cell) | < 10 |
| MAXF MCF7X | Mamma | 175,332 |
| MEFX 514 | Melanom | 182,074 |
| MEFX 989 | Melanom | 87,671 |
| OVXF 1023 | Ovar | 186,070 |
| PRXF PC3M | Pankreas | 529,431 |
| RXF 486 | Renal | 115,407 |
| RXF 1220 | Renal | 175,009 |
| RXF 944LX | Renal | 922,439 |

Die Kolonkarzinome CXF 280, CXF 609 und das Lungenkarzinom LXFS 650 wiesen eine besondere Empfindlichkeit gegen PHMB auf, die größer als eine log-Stufe gegenüber den anderen empfindlichen Tumorarten war. Auch das dritte untersuchte Kolonkarzinom CXF HT29X gehörte zu den empfindlichsten Tumoren.

**Beispiel 4**

Untersuchung der antitumoralen Wirksamkeit von PHMB $M_w$ 2.800:
Die Untersuchung wurde unter Verwendung der gemäß Beispiel 2 hergestellten Lösung in der gemäß Beispiel 3 beschriebenen Weise durchgeführt.
Bei dieser Untersuchung wurden Ergebnisse erhalten, die mit den gemäß Beispiel 3 beschriebenen Ergebnissen vergleichbar waren.

**Beispiel 5**

Zur Herstellung einer weiteren erfindungsgemäßen Lösung wurde Beispiel 1 wiederholt mit der Ausnahme, daß anstelle des Polyethylenglycol, $M_w$ 4.000, Wasser verwendet wurde. Die erhaltene Lösung hatte folgende Zusammensetzung:

| | |
|---|---|
| PHMB-Hydrochlorid, $M_w$ 2.800 | 20 Gew.-% |
| Wasser | 80 Gew.-%. |

Diese Lösung wurde in der gleichen Weise wie in Beispiel 3 untersucht, wobei mit den in Beispiel 3 beschriebenen Ergebnissen vergleichbare Ergebnisse erhalten wurden.

**Beispiel 6**

Zur Herstellung einer erfindungsgemäßen Lösung wurde Beispiel 5 wiederholt mit der Ausnahme, daß anstelle des PHMB-Hydrochlorids das entsprechende PHMB eingesetzt wurde.

**Beispiel 7**

Aus PHMB mit einem Molekulargewicht von 3.500, Polyethylenglycol 4.000 und Wasser, wurde in der gleichen Weise wie in Beispiel 1 beschrieben durch Vermischen der Bestandteile eine erfindungsgemäße Lösung der folgenden Zusammensetzung hergestellt:

| Poly(hexamethylen)biguanid-Hydrochlorid, $M_w$ 3.500 | 20 Gew.-% |
|---|---|
| Polyethylenglycol, $M_w$ 4.000 | 1,0 Gew.-% |
| Wasser | 79 Gew.-% |

Das angewandte Poly(hexamethylen)biguanid-Hydrochlorid wurde in an sich bekannter Weise durch fraktionierte Filtration aus dem handelsüblichen Poly(hexamethylen)biguanid-Produkt Vantocil® IB, Cosmocil CQ oder Arlagard E der Firma ICI abgetrennt. Vantocil IB, Cosmocil CQ bzw. Arlagard E ist eine wässrige Lösung, die als Wirkstoff 20 % Poly(hexamethylen)biguanid-Hydrochlorid enthält.

**Beispiel 8**

Zur Herstellung einer weiteren erfindungsgemäßen Lösung wurde Beispiel 7 wiederholt mit der Ausnahme, daß anstelle des dort verwendeten PHMB-Hydrochlorids mit $M_w$ 3.500 ein in gleicher Weise hergestelltes PHMB-Hydrochlorid mit $M_w$ 5.000 angewandt wurde.

**Beispiel 9**

Aus PHMB mit einem mittleren Molekulargewicht $M_w$ 4.000 und Wasser wurde durch Vermischen derselben eine erfindungsgemäße Lösung der folgenden Zusammensetzung hergestellt:

| PHMB, $M_w$ 4.000 | 4,5 Gew.-% |
|---|---|
| Wasser | 95,5 Gew.-%. |

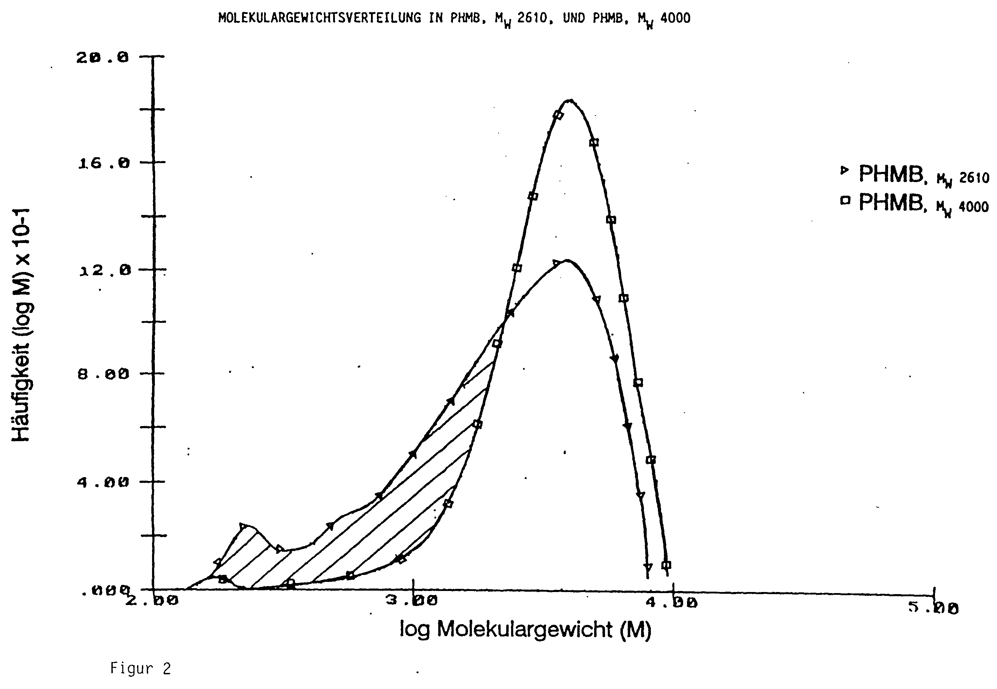

Das angewandte PHMB wurde gemäß der in Beispiel 7 beschriebenen Weise erhalten.
Aus Figur 2 ist die Molekulargewichtsverteilung von PHMB, $M_w$ 4.000, im Vergleich zum handelsüblichen PHMB, $M_w$ 2.600, ersichtlich.

**Beispiel 10**

Aus PHMB mit einem mittleren Molekulargewicht $M_w$ 5.000 und Wasser wurde durch Vermischen derselben eine erfindungsgemäße Lösung der folgenden Zusammensetzung hergestellt:

| PHMB, $M_w$ 5.000 | 4,0 Gew.-% |
|---|---|
| Wasser | 96 Gew.-%. |

Das angewandte PHMB wurde gemäß der im Beispiel 7 beschriebenen Weise erhalten.

**Beispiel 11**

Aus PHMB mit einem mittleren Molekulargewicht $M_w$ 4.500 und Wasser wurde durch Vermischen derselben eine erfindungsgemäße Lösung der folgenden Zusammensetzung hergestellt:

| PHMB, $M_w$ 4.500 | 4,2 Gew.-% |
|---|---|
| Wasser | 95,8 Gew.-%. |

Das angewandte PHMB wurde gemäß der im Beispiel 7 beschriebenen Weise erhalten.

**Beispiel 12**

Aus PHMB mit einem mittleren Molekulargewicht $M_w$ 4.000 und Wasser wurde durch Vermischen derselben eine erfindungsgemäße Lösung der folgenden Zusammensetzung hergestellt:

| PHMB, $M_w$ 4.000 | 20 Gew.-% |
|---|---|
| Wasser | 80 Gew.-%. |

Das angewandte PHMB wurde gemäß der im Beispiel 7 beschriebenen Weise hergestellt.

**Beispiel 13**

Zur Herstellung einer intravenös zu verabreichenden Lösung wurde die in Beispiel 11 hergestellte Lösung mit lactatfreier Ringerlösung auf eine Endkonzentration an PHMB, $M_w$ 4.500, von 0,0012 Gew.-% verdünnt.

**Beispiel 14**

Zur Herstellung einer weiteren intravenös zu verabreichenden Lösung wurde die in Beispiel 11 hergestellte Lösung mit lactatfreier Ringerlösung auf eine Endkonzentration an PHMB, $M_w$ 4.500, von 0,01 Gew.-% verdünnt.

**Beispiel 15**

Zur Herstellung einer weiteren erfindungsgemäßen intravenös zu verabreichenden Lösung wurde die in Beispiel 10 hergestellte Lösung mit 0,9 %iger Kochsalzlösung auf eine Endkonzentration an PHMB, $M_w$ 5.000, von 0,005 Gew.-% verdünnt.

**Patentansprüche**

1. Verwendung von Poly(hexamethylen)biguanid oder eines Salzes desselben zur Herstellung eines Arzneimittels zur Behandlung von Tumorerkrankungen.

2. Verwendung nach Patentanspruch 1, **dadurch gekennzeichnet, daß** das Poly(hexamethylen)biguanid eine mittlere Molekulargewichtsverteilung bis 15.000, vorzugsweise 1.000 bis 8.000, insbesondere 1.700 bis 5.000, besitzt.

3. Verwendung nach Patentanspruch 1 oder 2, **dadurch gekennzeichnet, daß** das Poly(hexamethylen)biguanid zusammen mit einem die Oberflächenspannung erniedrigenden Tensid verwendet wird.

4. Verwendung nach Patentanspruch 3, **dadurch gekennzeichnet, daß** das Tensid Polyethylenglycol ist.

5. Verwendung nach Patentanspruch 4, **dadurch gekennzeichnet, daß** das Verhältnis von Poly(hexamethylen)biguanid zu Polyethylenglycol im Bereich von 6:1 bis 24:1 liegt.

6. Verwendung nach Patentanspruch 5, **dadurch gekennzeichnet, daß** das Verhältnis im Bereich von 12:1 bis 22:1 liegt.

7. Verwendung nach einem oder mehreren der Patentansprüche 1 bis 6, **dadurch gekennzeichnet, daß** das Poly(hexamethylen)biguanid in lactatfreier Ringerlösung oder Kochsalzlösung angewandt wird.

8. Verwendung nach einem oder mehreren der Patentansprüche 1 bis 7, **dadurch gekennzeichnet, daß** das Poly(hexamethylen)biguanid in einer Konzentration von 0,0001 bis 1 Gew.-% angewandt wird.

9. Verwendung nach Patentanspruch 8, **dadurch gekennzeichnet, daß** die Konzentration 0,0005 bis 0,1 Gew.-% beträgt.

10. Verwendung nach Patentanspruch 9, **dadurch gekennzeichnet, daß** die Konzentration 0,001 bis 0,04 Gew.-% beträgt.

**EP 0 819 434 B1**

**Claims**

1. Use of poly(hexamethylene)biguanide or a salt thereof in the production of a medicament for treating tumour diseases.

2. Use according to patent claim 1, **characterised in that** the poly(hexamethylene)biguanide has a mean molecular weight distribution of up to 15,000, preferably 1,000 to 8,000 and especially of 1,700 to 5,000.

3. Use according to patent claim 1 or 2, **characterised in that** the poly(hexamethylene)biguanide is used together with a tenside which lowers the surface tension.

4. Use according to patent claim 3, **characterised in that** the tenside is polyethylene glycol.

5. Use according to patent claim 4, **characterised in that** the ratio of poly(hexamethyllene)biguanide to polyethylene glycol is in the range from 6:1 to 24:1.

6. Use according to patent claim 5, **characterised in that** the ratio is in the range from 12:1 to 22:1.

7. Use according to one or more of patent claims 1 to 6, **characterised in that** the poly(hexamethylene)biguanide is used in a lactate-free ringer solution or cooking salt solution.

8. Use according to one or more of patent claims 1 to 7, **characterised in that** the poly(hexamethylene)biguanide is used in a concentration of 0.0001 to 1% by weight.

9. Use according to patent claim 8, **characterised in that** the concentration amounts to 0.0005 to 0.1% by weight.

10. Use according to patent claim 9, **characterised in that** the concentration amounts to 0.001 to 0.04% by weight.


**Revendications**

1. Utilisation du poly(hexaméthylène)biguanide ou d'un de ses sels pour la préparation d'un produit thérapeutique pour le traitement de maladies tumorales.

2. Utilisation selon la revendication 1, **caractérisée en ce que** le poly(hexaméthylène)biguanide présente une répartition de masse molaire moyenne jusqu'à 15000, de préférence de 1000 à 8000, en particulier de 1700 à 5000.

3. Utilisation selon la revendication 1 ou 2, **caractérisée en ce qu'**on utilise le poly(hexaméthylène)biguanide conjointement avec un agent de surface réduisant la tension superficielle.

4. Utilisation selon la revendication 3, **caractérisée en ce que** l'agent de surface et le polyéthylèneglycol.

5. Utilisation selon la revendication 4, **caractérisée en ce que** le rapport du poly(hexaméthylène)biguanide au polyéthylèneglycol est dans le domaine de 6:1 à 24:1.

6. Utilisation selon la revendication 5, **caractérisée en ce que** le rapport se trouve dans le domaine de 12:1 à 22:1.

7. Utilisation selon une ou plusieurs des revendications 1 à 6, **caractérisée en ce qu'**on emploie le poly(hexaméthylène)biguanide dans une solution de Ringer sans lactate ou une solution de chlorure de sodium.

8. Utilisation selon une ou plusieurs des revendications 1 à 7, **caractérisée en ce qu'**on emploie le poly(hexaméthylène)biguanide en une concentration de 0,0001 à 1 % en masse.

9. Utilisation selon la revendication 8, **caractérisée en ce que** la concentration est de 0,0005 à 0,1 % en masse.

10. Utilisation selon la revendication 9, **caractérisée en ce que** la concentration est de 0,001 à 0,04 % en masse.

**Figur 1:** **Wirkung von Polyhexamethylenbiguanid (PHMB) auf das Wachstum verschiedener Tumorarten**

IC70 µg/ml PHMB

Kolon    Lunge (small cell)    Mamma    Melanom    Ovar    Pankreas    Renal    Lunge

**Tumorart**

EP 0 819 434 B1

MOLEKULARGEWICHTSVERTEILUNG IN PHMB, $M_W$ 2610, UND PHMB, $M_W$ 4000

Figur 2

EP 0 819 434 B1